Europäisches Patentamt

European Patent Office

Office européen des brevets

(11) Publication number: 0 400 547
A1

# EUROPEAN PATENT APPLICATION

(21) Application number: 90110103.0

(51) Int. Cl.⁵: **A61K 31/23**

(22) Date of filing: 28.05.90

(30) Priority: 02.06.89 US 360966
29.12.89 US 458858

(43) Date of publication of application:
05.12.90 Bulletin 90/49

(84) Designated Contracting States:
AT BE CH DE DK ES FR GB GR IT LI LU NL SE

(71) Applicant: ABBOTT LABORATORIES
CHAD-0377, AP6D/2, One Abbott Park Road
Abbott Park, Illinois 60064-3500(US)

(72) Inventor: Shaw, Howard Laurence
1329 East Canton Court
Deerfield, Illinois 60015(US)
Inventor: Leathem, William D.
541 Beck Road
Lindenhurst, Illinois 60046(US)

(74) Representative: Modiano, Guido et al
MODIANO, JOSIF, PISANTY & STAUB
Modiano & Associati Via Meravigli, 16
I-20123 Milano(IT)

(54) Parenteral nutrition product.

(57) Parenteral nutritional compositions are described that contain a source of gamma-linolenic acid. A method of parenterally administering these compositions is also disclosed.

EP 0 400 547 A1

## PARENTERAL NUTRITION PRODUCT

### FIELD OF THE INVENTION

The present invention relates to a composition and method for the parenteral administration of fatty acids. More particularly, this invention relates to gamma-linolenic acid containing lipid emulsions for parenteral administration.

### BACKGROUND OF THE INVENTION

Commercially available parenteral fat emulsions contain linoleic acid (18:2w6) as the principal fatty acid. Linoleic acid is converted to gamma-linolenic acid (18:3w6) with the aid of the enzyme, delta-6-desaturase. Gamma-linolenic acid is relatively rare in the human diet. Gamma-linolenic acid is found in human milk and in some oils such as borage oil. The conversion of linoleic acid to gamma-linolenic acid is the rate limiting step in the preparation of arachidonic acid and the prostaglandins, prostacyclin, $PGE_2$ and thromboxane. Synthesis of prostacyclin and $PGE_2$ is very desirable because they have a range of cytoprotective, antiaggretory, bronchodilation, vasodilation and anti-inflammatory properties.

Under certain conditions delta-6-desaturase is slow, absent or non-functioning in mammals. If linoleic acid is the primary nutritional fatty acid source, elevated levels of linoleic acid and an associated deficit of gamma-linolenic acid and other prostaglandin precursors can result. For example, gamma-linolenic acid production in neonates is low. Viral infections, saturated fats, aging, cancer, low zinc levels, alcohol, diabetes, Sjogren's syndrome and scleroderma also decrease the synthesis of gamma-linolenic acid and prostaglandins.

Thus, there is a need for a parenteral nutritional composition that avoids the effects of a sdeficit in the presence or function of delta-6-desaturase and increases the rate of formation of desirable prostaglandins.

### SUMMARY OF THE INVENTION

The present invention relates to a parenteral nutritional composition that contains gamma-linolenic acid as a component with diminished levels of linoleic acid.

More particularly, this invention relates to parenteral nutritional compositions that comprise from about 2 to about 30 weight percent fatty acids calculated as triglycerides, said fatty acids containing from about 1 to about 25 weight percent gamma-linolenic acid, and sufficient egg phosphatide, glycerin and water to prepare a lipid emulsion for parenteral administration. The compositions of the present invention can optionally contain fat soluble vitamins, as for example, vitamins A, D and E and L-carnitine. This invention also relates to a method of parenterally administering a therapeutically effective amount of a composition of the present invention to a mammal in need of such treatment.

### DETAILED DESCRIPTION OF THE INVENTION

Presently preferred parenteral nutritional compositions contain borage oil as a source of gamma-linolenic acid. Borage oil is extracted by conventional techniques such as low temperature expeller pressing, direct solvent extraction or supercritical fluid extraction from the mature dried seeds of Borago officionale L., an herbaceous annual of the plant family Boraginaceae. Borage oil contains approximately 20 to percent by weight of gamma-linolenic acid. The content of linoleic, gamma-linolenic and linolenic acids in borage oil, black currant seed oil, evening primrose oil and soybean oil is shown in TABLE 1.

2

TABLE 1

| FATTY ACID COMPOSITION (%) OF GAMMA-LINOLENIC ACID CONTAINING SEED OILS | | | | |
|---|---|---|---|---|
| FATTY ACIDS | BORAGE OIL | BLACKCURRANT SEED | EVENING PRIMROSE OIL | SOYBEAN OIL |
| Linoleic Acid 18:2w6 | 35-40 | 45-49 | 73-75 | 53-57 |
| Gamma-Linolenic Acid 18:3w6 | 21-25 | 15-19 | 7-10 | -- |
| Linolenic Acid 18:3w3 | -- | 13-15 | -- | 7-9 |

The parenteral nutritional compositions of the present invention can contain from about 2 to about 30 weight percent fatty acids calculated as triglycerides. The fatty acids generally include a source of linoleic acid such as soybean or safflower oil, or mixtures thereof, and a source of gamma-linolenic acid such as borage oil. Soybean, safflower and borage oils are comprised of long chain triglycerides which contain fatty acids of 12 to 26 carbon atoms in length. In a preferred embodiment, borage oil is present in an amount sufficient to yield gamma-linolenic acid in an amount of from about 1 to about 25 weight percent of the total weight of fatty acids present in the composition. The nutritional composition can also contain medium chain fatty acids of 6 to 12 carbon atoms in length or mixtures thereof.

A parenteral nutritional composition of the present invention can comprise the following amounts of the below enumerated ingredients:

| Ingredient | Weight Percent |
|---|---|
| Soybean oil | 0 - 30 |
| Borage oil | 0.4 - 3.0 |
| (gamma-linolenic acid) | (0.1 - 7.5) |
| Egg phosphatide | 1 - 2 |
| Glycerin | 2 - 3 |
| Water | 56 - 87 |

The compositions of the present invention can also contain sufficient quantities of fat or water soluble vitamins to meet minimum daily requirements. For example, the compositions of the present invention can contain up to 2300 IU/day of Vitamin A for pediatric patients and up to 3300 IU/day of Vitamin A for adult patients, up to 400 IU/day of Vitamin D, up to 50 IU/day of Vitamin E and from 0.4-1.0 g/L-carotine/day. Other vitamins and minerals can be added in amounts sufficient to satisfy the daily requirements of adult, pediatric and neonatal patients.

The gamma-linolenic acid enriched parenteral nutritional formulations of the present invention can be used to treat patients with a variety of conditions where patients have a low level of delta-6-desaturase activity. For example, they may be used to treat:

1. neonates with immature enzyme systems;
2. patients with cystic fibrosis;
3. critically ill patients with acute respiratory distress syndrome and/or sepsis;
4. diabetics;
5. patients or alcoholics with hepatic dysfunction; and
6. patients recuperating from surgery or other acute trauma.

Other conditions that may be treated with gamma-linolenic acid enriched parenteral treatment formulations are cancer, acquired immune deficiency syndrome (AIDS), autoimmune disorders, hypermetabolic disorders and cardiovascular diseases.

Representative compositions of the present invention include the following:

| Gamma-Linolenic Acid Enriched Lipid Emulsion 20% | | | |
|---|---|---|---|
| Ingredient | 3% GLA | 5% GLA | 7% GLA |
| Soybean oil | 17.6g | 16.0g | 14.4g |
| Borage oil | 2.4g | 4.0g | 5.6g |
| (Gamma-linolenic acid) | (0.6g) | (1.0g) | (1.4g) |
| Egg Phosphate | 1.2g | 1.2g | 1.2g |
| Glycerin | 2.5g. | 2.5g | 2.5g |

In a treatment method of the present invention, compositions of the present invention are generally administered parenterally to patients in amounts sufficient to provide 0.02 to 4.0 g/kg day of lipid. In a preferred embodiment, a formulation containing about 20 weight percent of a composition of the present invention is parenterally administered to a mammal or human patient.

The composition of the present invention may be prepared in accordance with known procedures in the art as illustrated by the following Examples which are not intended to limit the scope of the invention.

Example 1

Water for injection (approximately 1 L) is heated and protected by a nitrogen atmosphere. Egg phosphatide (12g) is added to approximately 600 ml of the water and dispersed by agitation at a temperature in the range of 50° to 90°C. Glycerin (25g) is filtered through an 0.8 micrometer membrane filter and added to the dispersion. A Manton-Gaulin homogenizer is used to finely divide the egg phosphatide and increase the degree of dispersion. The aqueous phosphatide dispersion is then filtered through a nylon or equivalent membrane of 0.45 micrometer porosity and the pH is adjusted to a range of 8.5 to 10.5 with sodium hydroxide. Soybean oil, winterized (17.6g) and borage oil, winterized (2.4g) are filtered through a 0.45 micrometer membrane, heated to a temperature in the range of 55° to 95°C and added to the egg phosphatide dispersion with agitation to form a coarse emulsion concentrate which is then homogenized using a Manton-Gaulin homogenizer at a pressure of 2000 to 8000 psi. The pH is then adjusted to a range of 8.5 to 9.5 if necessary with sodium hydroxide and the emulsion filtered through a nylon membrane of at least 0.8 micrometer porosity with sufficient surface area to provide minimum restriction of flow. The emulsion is then further homogenized in a Manton-Gaulin homogenizer, diluted to its desired concentration with sterile water and the pH adjusted to a range of 8.5 to 9.5 with sodium hydroxide.

Example 2

Adult male Sprague-Dawley rats weighing 275 ± 15g were parenterally administered a 20% composition containing a 1:1 mixture of soybean and safflower oils or a lipid emulsion containing 3%, 5% or 7% by weight of gamma-linolenic acid (GLA). Control rats in each group were unstressed, while stressed rats received a thermal injury (immersion of shaved dorsal surface of rat in boiling water for 15 seconds) followed by an intraperitoneal, nonlethal 1 ml dose (0.5 mg/kg) of endotoxin (Salmonella enteriditis) in 0.9% saline, to induce sepsis.

All rats were fluid resuscitated with an intraperitoneal injection of sterile lactated Ringers solution (2.5 ml/100g) followed by a four-hour intravenous infusion of 0.9% heparinized saline (10 U/ml) at a rate of 2.5 ml/hr.

Total intravenous nutrition was then delivered for three days through the left jugular vein. Each rat received 200 kcal/kg/day total calories, of which 40.2 kcal/kg/day were amino acids and 159.8 kcal/kg/day were non-protein calories (70% from dextrose and 30% from the lipid emulsion). After three days of total parenteral nutrition, the rats were sacrificed by exsanguination. Plasma samples were analyzed for total fatty acids present.

TABLE 2

| Total Plasma Fatty Acids (%) | | | | | | |
|---|---|---|---|---|---|---|
| Group | N[1] | Linoleic | gamma-Linolenic | DiHomo-gamma-Linolenic | Arachidonic | Linolenic |
| | | 18:2w6 | 18:3w6 | 20:3w6 | 20:4w6 | 18:3w3 |
| Unstressed Rats [2] | | | | | | |
| soybean:safflower | 10 | 43.3 ± 2.1 | 0 | 0 | 19.7 ± 1.6 | 0.9 ± 0.3 |
| 3% GLA | 7 | 34.5 ± 0.7 | 0.5 ± .1 | 0.08 ± .08 | 22.7 ± 0.5 | 1.7 ± 0.1 |
| 5% GLA | 7 | 36.3 ± 1.3 | 1.1 ± .3 | 0.73 ± .2 | 20.7 ± 1.1 | 1.7 ± 0.4 |
| 7% GLA | 9 | 32.8 ± 1.3 | 1.8 ± .2 | 0.60 ± .2 | 22.9 ± 0.9 | 1.6 ± 0.1 |
| Stressed Rats [2] | | | | | | |
| soybean:safflower | 7 | 34.1 ± 2.2 | 0.09 ± .06 | 0.4 ± .24 | 27.7 ± 1.6 | 1.15 ± 0.2 |
| 3% GLA | 8 | 28.1 ± 1.2 | 0.35 ± .14 | 0.3 ± .14 | 30.7 ± 1.2 | 0.7 ± 0.3 |
| 5% GLA | 8 | 29.6 ± 1 | 1.3 ± .2 | 0.63 ± .14 | 30.7 ± 1.6 | 1.6 ± 0.1 |
| 7% GLA | 8 | 28.4 ± 1.1 | 1.7 ± .08 | 0.99 ± .06 | 29.8 ± 1.4 | 1.4 ± 0.1 |

1. N = Number of animals
2. Values = mean ± S.E.M.

Example 3

Rats were maintained on total intravenous nutrition, as described in Example 2, and the isolated blood plasma was analyzed to determine the concentration of thromboxone $B_2$ (TXB2), 6-keto-prostaglandin $F_1$ alpha (KPGF), and bicyclic $PGE_2$ (BPGE) present.

The results are shown in TABLE 3.

TABLE 3

| Plasma Prostaglandin Concentration (PG/ml) | | | | | |
|---|---|---|---|---|---|
| Group | N[1] | TXB2 | KPGF | BPGE | TXB2/KPGF |
| Unstressed Rats [2] | | | | | |
| soybean:safflower | 10 | 1188 ± 225 | 190 ± 71 | 353 ± 22 | 11 ± 2 |
| 3% GLA | 7 | 1218 ± 291 | 135 ± 32 | 342 ± 37 | 14 ± 6 |
| 5% GLA | 7 | 1013 ± 254 | 311 ± 72 | 386 ± 57 | 5 ± 1 |
| 7% GLA | 9 | 828 ± 253 | 109 ± 22 | 311 ± 84 | 14 ± 7 |
| Stressed Rats [2] | | | | | |
| soybean:safflower | 7 | 1608 ± 231 | 213 ± 132 | 247 ± 25 | 24 ± 8 |
| 3% GLA | 8 | 1771 ± 199 | 320 ± 61 | 420 ± 99 | 7 ± 1 |
| 5% GLA | 8 | 1590 ± 175 | 543 ± 143 | 352 ± 42 | 8 ± 4 |
| 7% GLA | 8 | 1133 ± 211 | 208 ± 77 | 360 ± 75 | 11 ± 3 |

1. N = Number of animals
2. Values = mean ± S.E.M.

Example 4

Rats maintained on total intravenous nutrition, as described in Example 2, were monitored for changes in body weight during the studies. The results are shown in Table 4.

TABLE 4

| Group | N[1] | Body Weight Change (grams) |
|---|---|---|
| Unstressed Rats [2] | | |
| soybean:safflower | 10 | -7.0 ± 3.1 |
| 3% GLA | 7 | 0.3 ± 1.3 |
| 5% GLA | 8 | 2.2 ± 1.7 |
| 7% GLA | 9 | -2.7 ± 1.3 |
| Stressed Rats [2] | | |
| soybean: safflower | 7 | -14.5 ± 2.0 |
| 3% GLA | 8 | -7.2 ± 1.9 |
| 5% GLA | 8 | -13.8 ± 2.1 |
| 7% GLA | 8 | -11.1 ± 1.1 |

1. N = Number of animals
2. Values = mean ± S.E.M.

## Example 5

Rats maintained on total intravenous nutrition, as described in Example 2, were monitored for cumulative nitrogen balance by collecting 24-hour urine samples over the three-day period of intravenous nutrition. The nitrogen balance was determined as the difference between total urinary nitrogen and total dietary nitrogen intake (1. 5g of N/kg/day); the sum of the daily nitrogen balances was reported as the cumulative nitrogen balance.

The results are shown in TABLE 5.

TABLE 5

| Group | N[1] | Cumulative Nitrogen Balance (mg/day) |
|---|---|---|
| Unstressed Rats [2] | | |
| soybean:safflower | 10 | 357 ± 34 |
| 3% GLA | 7 | 351 ± 44 |
| 5% GLA | 8 | 309 ± 46 |
| 7% GLA | 9 | 348 ± 31 |
| Stressed Rats [2] | | |
| soybean: safflower | 7 | -85 ± 41 |
| 3% GLA | 8 | -115 ± 28 |
| 5% GLA | 8 | -66 ± 30 |
| 7% GLA | 8 | -127 ± 17 |

1. N = Number of animals
2. Values = mean ± S.E.M.

The foregoing specification, including the specific embodiments and Examples, is intended to be illustrative of the present invention and is not to be taken as limiting. Numerous other variations and modifications can be made without departing from the true spirit and scope of the present invention.

**Claims**

1. A parenteral nutritional composition that comprises from about 2 to about 30 weight percent fatty acids calculated as triglycerides where from about 1 to about 25 weight percent of the fatty acids is gamma-linolenic acid.

2. The composition according to Claim 1 wherein the gamma-linolenic acid is derived from borage oil.

3. The composition according to Claim 1 that further comprises fat soluble vitamins or L-carnitine.

4. A composition according to Claim 1 for parenteral administration to a mammal.

5. A composition for parenteral administration to a mammal comprising about 2 to about 30 weight percent fatty acids calculated as triglycerides, where from about 1 to about 25 weight percent of said fatty acids is gamma-linolenic acid, to provide parenteral nutrition to said mammal.

6. A formulation for administration to a mammal comprising about 20 percent by weight of the composition according to Claim 5.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 084 172 (ROUSSEL-UCLAF) * Claims 1,15,16,19 * | 1,3-5 | A 61 K 31/23 |
| X | DE-A-2 749 492 (BIO-OIL RESEARCH LTD) * Claim 6 * | 2 | |

TECHNICAL FIELDS SEARCHED (Int. Cl.5)

A 61 K 31

The present search report has been drawn up for all claims

| Place of search | Date of completion of the search | Examiner |
|---|---|---|
| THE HAGUE | 07-09-1990 | LEHERTE C.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)